# EUROPEAN PATENT SPECIFICATION

(11) **EP 2 349 187 B1**
(45) Date of publication and mention of the grant of the patent: **06.09.2017**
(21) Application number: 09748820.9
(22) Date of filing: 13.11.2009
(51) Int. Cl.: A61K 8/19, A61K 8/21, A61K 8/24, A61K 8/365, A61Q 11/00

(54) **ORAL CARE COMPOSITION**
MUNDPFLEGEMITTEL
COMPOSITIONS DE PROTECTION ORALE

(30) Priority: 28.11.2008 EP 08170278
(43) Date of publication of application: 03.08.2011
(73) Proprietor: Unilever PLC, London Greater London EC4Y 0DY (GB); Unilever N.V., 3013 AL Rotterdam (NL)
(72) Inventor: INGEGNERI, Irene, I-26841 Casalpusterlengo (Lo) Lodi (IT)
(74) Representative: Tansley, Sally Elizabeth
(86) International application number: PCT/EP2009/065174
(87) International publication number: WO 2010/060816

(56) References cited:
- EP-A- 0 342 746
- WO-A-2007/122146

## Description

The present invention relates to an oral care composition comprising hydroxyapatite and a source of fluoride. The invention also relates to the stabilisation of the fluoride in such products.

US 4,244,931 (Monsanto Co., 1981) discloses dentifrice abrasives comprising dicalcium phosphate dihydrate having improved fluoride stability. US 5,037,636 (Monsanto Co., 1991) discloses dentifrice abrasives comprising dicalcium phosphate dihydrate and small amounts of zinc salts having improved fluoride stability. WO 03/059303 (P&G Co., 2003) discloses oral care compositions comprising complexes of amorphous calcium phosphates and phosphopeptides, with a specific pH and buffer system, having enhanced fluoride stability. EP 0342746 discloses oral compositions comprising particulate hydroxyapatite and fluorides, having good stability. In a first aspect of the present invention, there is provided an oral care composition comprising particulate hydroxyapatite, a source of fluoride, a silica based abrasive and potassium citrate. In a second aspect of the present invention, there is provided a method of improving the stability of fluoride in an oral care composition also comprising a silica based abrasive and particulate hydroxyapatite, said method comprising the inclusion of a potassium citrate in the oral care composition.

In a third aspect of the present invention, there is provided a method of manufacture of an oral care composition according to the first aspect of the invention.

In a fourth aspect of the present invention, there is provided the use of particulate hydroxyapatite, a source of fluoride, a silica based abrasive and potassium citrate in the manufacture of an oral care composition for the treatment of the teeth in the oral cavity.

The treatment referred to in the fourth aspect of the invention may be for anticaries benefits, for remineralisation of the teeth, or for reducing hypersensitivity.

The oral care compositions of the present invention are typically used to treat the teeth in the human mouth. The composition may be a mouthwash or a gum, but is more typically a toothpaste.

The hydroxyapatite used in the present invention may be of any of the forms suitable for inclusion in the composition in which it is incorporated. Finely divided hydroxyapatite is particularly suitable, such material having been previously used as a dentifrice abrasive (see CA-A-999,238, US-A-4,634,589 and US-A-4, 327,079). The average particle size of such finely divided hydroxyapatite is usually in the range from about 0.5 micron to about 15 microns, preferably from 1 to 10 and particularly from about 2 to about 5 microns.

Preferred particulate hydroxyapatites are synthetic and are of high purity consisting of at least 92% by weight of Ca₁₀(PO₄)₆(OH)₂. The remainder will comprise mainly bound water (typically 6% by weight maximum) and a minor amount of calcium carbonate.

Preferred particulate hydroxyapatites have needle-shaped primary crystallites, typically of length from 100 to 200 nm and width and thickness from 10 to 20 nm. These primary crystallites are typically agglomerated into larger particles having the particle size distributions indicated above.

The particulate hydroxyapatite is typically incorporated at from 0.1 to 20% by weight, preferably at from 0.25 to 10% and more preferably at from 0.5 to 5% by weight of the composition of which it is a part.

The source of fluoride is typically a complex fluoride salt such as sodium monofluorophosphate (SMFP) or potassium monofluorophoshate. Complex fluoride salts should be understood to be salts in which the fluorine atom is bound to another atom in the anion of the salt, rather than being present as a free fluoride anion. SMFP is the most preferred source of fluoride.

The fluoride source is preferably present at such a level as to provide fluoride at from 100 to 2000 ppm, and preferably from 900 to 1500 ppm in the composition.

When the source of fluoride is SMFP, it is typically incorporated at from 0.1% to 10%, preferably at from 0.25 to 5% and more preferably at from 0.5 to 2.5% by weight of the composition of which it is a part.

The source of potassium cations is potassium citrate, which is also a source of citrate ions (*vide infra*).

The source of citrate anions is typically a simple salt. Typically the citrate ion is present in its tri-basic form; however, acid salts may be contemplated. Potassium citrate is the source of citrate ions because this is also a source of potassium ions (*vide supra*).

Potassium citrate, i.e. tripotassium citrate, is incorporated in compositions of the invention typically at from 0.1 to 30%, preferably at from 0.5 to 20% and more preferably at from 1 to 10% by weight of the composition of which it is a part.

As well as stabilising the fluoride, potassium citrate may enhance the desensitising benefit of the composition.

A preferred additional component is a source of zinc ions, in particular zinc citrate.

When included, zinc citrate is typically present at from 0.1 to 10%, preferably from 0.5 to 5%, and more preferably 1 to 3%, by weight of the composition of which it is part.

In preferred embodiments, compositions of the invention comprise one or more types of silica. A thickening silica is one such silica, by which is meant a silica that causes an increase in viscosity of the composition.

An abrasive silica is another component, such silicas being particulate materials that are relative hard and abrasive.

The composition may contain other particulate abrasive materials such as alumina, dicalcium phosphate, calcium pyrophosphate, trimetaphosphate, or insoluble hexametaphosphate.

The total amount of particulate abrasive material, including the hydroxyapatite and any abrasive silica (*vide infra*) present, is typically from 3 to 60% by weight of the composition.

In addition to a source of fluoride, the composition may comprise other anti-caries agents such as sodium trimetaphosphate or casein.

The toothpaste composition according to the invention may comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
Humectants such as glycerol, sorbitol, propyleneglycol, xylitol, lactitol etc.;
binders and thickeners such as sodium carboxymethyl-cellulose, hydroxyethyl cellulose (Natrosol®), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the toothpaste composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

Except in the operating and comparative examples, or where otherwise explicitly indicated, all numbers in this description indicating amounts of material ought to be understood as modified by the word 'about'.

The term 'comprising' is meant not to be limiting to any subsequently stated elements but rather to encompass non-specified elements of major or minor functional importance. In other words the listed steps, elements or options need not be exhaustive. Whenever the words 'including' or 'having' are used, these terms are meant to be equivalent to 'comprising' as defined above.

Embodiments according to the invention shall now be discussed with reference to the following non-limiting examples.

### Examples

Examples according to the invention are indicated by numbers and comparative examples are indicated by letter. Amounts given are percentages by weight, unless otherwise indicated.

The toothpaste compositions indicated in Table 1 were prepared.

**Table 1**

| | **Example** | | | |
|---|---|---|---|---|
| | **1** | **A** | **2** | **B** |
| | | | | |
| Water | 25.5 | 30.9 | 24.1 | 29.8 |
| Sorbitol (70% aqu.) | 45 | 45 | 45 | 45 |
| Sodium saccharin | 0.2 | 0.2 | 0.2 | 0.2 |
| PEG 32 | 2 | 2 | 2 | 2 |
| Trisodium phosphate | -- | -- | 1.1 | 1.1 |
| Tocopheryl acetate | 0.1 | 0.1 | 0.1 | 0.1 |
| Triclosan | -- | -- | 0.3 | -- |
| Potassium Citrate | 5.4 | -- | 5.4 | -- |
| Hydroxyapatite (1) | 2 | 2 | 2 | 2 |
| Thickening silica (2) | 7 | 7 | 7 | 7 |
| Sorbosil AC77 (3) | 6 | 6 | 6 | 6 |
| SMFP | 1.1 | 1.1 | 1.1 | 1.1 |
| Zinc Citrate | 2 | 2 | 2 | 2 |
| Sodium lauryl sulphate | 1.8 | 1.8 | 1.8 | 1.8 |
| Flavour | 1.2 | 1.2 | 1.2 | 1.2 |
| CMC (4) | 0.7 | 0.7 | 0.7 | 0.7 |

| | | | | |
|---|---|---|---|---|
| 1. *Ex* Budenheim. 2. Tixosil 43, ex Ineos. 3. Sorbosil AC77, ex Ineos. 4. Carboxymethylcellulose: CMC 9H, *ex* Dansico. | | | | |

The toothpaste compositions indicated in Table 1 were placed on storage at 40°C for two or three months. The level of fluoride in the compositions was monitored, measurements being made using gas chromatography, following conversion of the fluoride into trimethylfluorosilane by reaction with trimethylchlorosilane under acidic conditions. The results indicated in Table 2 were obtained.

**Table 2**

| **Storage** | **Fluoride level (ppm)** | | | |
|---|---|---|---|---|
| | **Example** | | | |
| | **1** | **A** | **2** | **B** |
| | | | | |
| For 2 months | -- | -- | 1412 | 1177 |
| For 3 months | 1208 | 1091 | -- | -- |

It is noteworthy that the compositions comprising potassium citrate have higher remaining fluoride levels than the corresponding compositions not comprising potassium citrate after storage.

It is also noteworthy that the long term stabilising effect of the potassium citrate is particularly important at higher temperatures. Table 3 illustrates this point for Example 2 vs. Comparative Example B.

**Table 3**

| **Storage** | **Fluoride level (ppm)** | | |
|---|---|---|---|
| | Example | | Percentage |
| | **2** | B | (B of 2) |
| **At 5°C** | | | |
| For 2 months | 1502 | 1412 | 94.0 |
| **At 25°C** | | | |
| For 2 months | 1485 | 1370 | 92.3 |
| **At 40°C** | | | |
| For 2 months | 1412 | 1177 | 83.4 |

## Claims

1. An oral care composition comprising from 0.5 to 5 wt% of the composition of particulate hydroxyapatite, a source of fluoride, potassium citrate, and a silica based abrasive.

2. An oral care composition according to claim 1 or claim 2, comprising a source of zinc cations.

3. An oral care composition according to claim 2, wherein zinc citrate is the source of zinc cations.

## Patentansprüche

1. Mundpflegezusammensetzung, umfassend 0,5 bis 5 Gew.-% der Zusammensetzung an partikelförmigem Hydroxyapatit, eine Quelle von Fluorid, Kaliumcitrat und ein abrasives Mittel auf Basis von Siliziumdioxid.

2. Mundpflegezusammensetzung nach Anspruch 1, umfassend eine Quelle von Zinkkationen.

3. Mundpflegezusammensetzung nach Anspruch 2, wobei Zinkcitrat die Quelle von Zinkkationen ist.

## Revendications

1. Composition de soin oral comprenant de 0,5 à 5 % en masse de la composition d'hydroxyapatite particulaire, une source de fluorure, du citrate de potassium et un abrasif à base de silice.

2. Composition de soin oral selon la revendication 1 ou la revendication 2, comprenant une source de cations de zinc.

3. Composition de soin oral selon la revendication 2, dans laquelle le citrate de zinc est la source de cations de zinc.
